# EUROPÄISCHE PATENTSCHRIFT

(11) **EP 4 392 114 B1**
(45) Veröffentlichungstag und Bekanntmachung des Hinweises auf die Patenterteilung: **10.09.2025**
(21) Anmeldenummer: 23729986.2
(22) Anmeldetag: 24.05.2023
(51) Int. Cl.: A61M 25/10, A61M 5/178

(54) **INFLATIONSVORRICHTUNG ZUR INFLATION EINES BALLONKATHETERS**
INFLATION DEVICE FOR INFLATING A BALLOON CATHETER
DISPOSITIF DE GONFLAGE POUR GONFLER UN CATHÉTER À BALLONNET

(30) Priorität: 25.05.2022 DE 102022113211
(43) Veröffentlichungstag der Anmeldung: 03.07.2024
(73) Patentinhaber: B. Braun Melsungen AG, 34212 Melsungen (DE)
(72) Erfinder: FULDA, Felix, 12059 Berlin (DE); STOLLE, Andreas, 12101 Berlin (DE)
(74) Vertreter: Winter, Brandl - Partnerschaft mbB
(86) Internationale Anmeldenummer: PCT/EP2023/063973
(87) Internationale Veröffentlichungsnummer: WO 2023/227686

(56) Entgegenhaltungen:
- WO-A1-2014/023250
- WO-A1-2016/152841
- WO-A1-99/27981
- US-A1- 2015 141 915

## Beschreibung

### Technisches Gebiet

Die vorliegende Offenbarung betrifft eine Inflationsvorrichtung zur Inflation eines Ballonkatheters zur Angioplastie oder zur Inflation anderer medizintechnischer Produkte, die auf der Technik des Ballonkatheters basieren.

Als Angioplastie wird ein Behandlungsverfahren zur (minimalinvasiven) Aufweitung von verengten oder verschlossenen Blutgefäßen bezeichnet, bei dem ein Ballonkatheter in das Blutgefäß eingeführt und das Blutgefäß durch Aufdehnen des Ballonkatheters, durch eine sogenannte Ballondilatation, aufgeweitet wird. Üblicherweise erfolgt die Angioplastie unter Röntgenkontrolle, bei der über den Ballonkatheter ein Kontrastmittel in das Blutgefäß injiziert wird, um eine verengte oder verschlossene Stelle des Blutgefäßes sowie eine Lage des Ballonkatheters im Blutgefäß beim Röntgen erkennen zu können.

Im Stand der Technik wird zur Angioplastie eine manuelle Inflationsspritze eingesetzt, mit der zum einen mittels (manuellem) Drehen eines Spritzenkolbens der Inflationsspritze ein Druckaufbau von bis zu 30 bar, etwa über ein Gewinde und eine hohe Übersetzung, sowie zum anderen (ohne Drehen des Spritzenkolbens) eine schnelle Druckentlastung, etwa über eine Entsperrung als Ring oder Hebel, erfolgen kann.

Dabei führt der manuell eingestellte (Über-)Druck in der Inflationsspritze zu einer entsprechenden Inflation des Ballonkatheters bzw. einem entsprechenden Durchmesser des Ballonkatheters. Ein Zusammenhang zwischen dem in der Inflationsspritze (und somit im Ballonkatheter) eingestellten Druck und dem sich daraus ergebenden Durchmesser kann in einem, üblicherweise in Papierform vorliegenden, Diagramm, einem sogenannten Compliance Chart, abgelesen werden, so dass ein Anwender einen erforderlichen Druck einem Ziel-Durchmesser entsprechend abschätzen und einstellen kann. Manchmal liegen die Informationen über den Zusammenhang von Druck im Ballonkatheter und Durchmesser des Ballonkatheters auch in Form einer Tabelle vor, einer sogenannten Compliance Table. In diesem Fall müssen die benötigten Werte gegebenenfalls aus den angegebenen Werten interpoliert werden. Zudem muss der Ballonkatheter vor der Inflation entlüftet werden, um das Vorhandensein von Luft in dem Ballonkatheter beim Druckaufbau auszuschließen. Dazu wird die Inflationsspritze an den Ballonkatheter angeschlossen und aufgezogen, wodurch ein Unterdruck in der Inflationsspritze erzeugt und die Luft aus dem Ballonkatheter herausgesaugt wird.

Nachteilig an der manuellen Einstellung ist jedoch, dass es, insbesondere beim Druckaufbau, zu einer Fehlanwendung in mehreren Hinsichten kommen kann: So können oberhalb eines zulässigen Bereichs liegende Drücke durch eine fehlende Möglichkeit der Druckbegrenzung erzeugt werden. Ferner kann es zu Ablesefehlern an einem Manometer zur (analogen) Druckanzeige oder an dem Diagramm kommen, was wiederum zu fehlerhaft eingestellten Drücken führt. Bei der Verwendung von Compliance Tables kann es außerdem zu Fehlern bei der Interpolation kommen.

Weiter ist es aus dem Stand der Technik bekannt, sensorisch zu überwachen, wie viel Fluid dem Ballonkatheter aus der Spritze zugeführt wird. Beispielsweise offenbart die Druckschrift US 2007 / 0 213 656 A1 eine Vorrichtung zum Erfassen und Ausgeben von Daten bei der Inflation eines aufweitbaren Bauteils, wie einem Ballonkatheter, mittels Fluidzufuhr aus einem Reservoir, wie einer Spritzenkammer. Die Vorrichtung weist Sensoren auf, mit denen der Druck, die Durchflussrate und/oder das Volumen des Fluids zum Aufweiten des Bauteils erfasst werden kann. Zudem weist die Vorrichtung einen Indikator auf, der den Druck, die Durchflussrate und/oder das Volumen qualitativ oder quantitativ anzeigt.

Zudem ist es aus der US2015/0141915 A1 bekannt, an einer Inflationsvorrichtung eine Kennzeichnung, in Form eines Barcodes oder QR-Codes vorzusehen, um in der Kennzeichnung gespeicherte oder damit verlinkte Informationen an einer portablen Displayeinheit auslesen zu können.

Weiter ist es aus der WO 2016/152841 A1 bekannt, eine Inflationsvorrichtung mit einer Spritze und einem Sensor zur Erfassung eines Fülldrucks in der Spritze vorzusehen, um einen Ballondurchmesser des entsprechenden Ballons berechnen zu können.

Darüber hinaus wird im Stand der Technik das Kontrastmittel vor der Angioplastie manuell in die Inflationsspritze gefüllt. Dazu wird das in unverdünnter Form vorliegende Kontrastmittel so weit verdünnt, bis ein gewünschtes Mischungsverhältnis für die Verwendung in der Inflationsspritze erhalten wird, da sich das Mischungsverhältnis auf die röntgendichten Eigenschaften auswirkt. Danach wird die Inflationsspritze mit dem verdünnten Kontrastmittel aufgezogen, bis eine erforderliche Menge an Kontrastmittel in der Inflationsspritze aufgenommen ist, die je nachdem, ob es sich beispielsweise um eine Angioplastie (perkutane transluminale Angioplastie, PTA) in peripheren Gefäßen oder eine Koronarangioplastie (perkutane transluminale Koronarangioplastie, PTCA) handelt, auch unterschiedlich sein kann. Beim Abbau des Unterdrucks füllt sich das vakuumierte Lumen des Ballonkatheters mit dem in der Inflationsspritze enthaltenen Kontrastmittelgemisch. Dabei kann eine zu große Menge an Kontrastmittel sich nachteilig auf eine ausreichende Entlüftung des Ballonkatheters vor der Angioplastie auswirken oder zu einer Verlängerung der Entleerung des Ballonkatheters nach der Angioplastie und einer damit einhergehenden Komplikation eines Entfernens des Ballonkatheters führen. Somit kann bei der manuellen Befüllung der Inflationsspritze mit Kontrastmittel eine falsche gewählte oder falsche eingestellte Menge an Kontrastmittel zu Fehlanwendungen führen.

Wenn ein Ballonkatheter im Patienten inflatiert wird, führt dies zu einer temporären Blockierung des Blutgefäßes. Daher ist unbedingt darauf zu achten, dass die Inflationszeit nicht zu lange wird, da dies unter anderem zu Schmerzen beim Patienten führen kann, der bei solch einem Eingriff bei vollem Bewusstsein ist. Auch kann eine zu lange Blockierung des Gefäßes zu Nekrosen führen. Unverdünntes Kontrastmittel ist deutlich zäher als Wasser, sodass in der Regel das Kontrastmittel so weit verdünnt wird, wie es im Röntgenbild noch erkennbar gegenüber dem umgebenden Gewebe kontrastiert, aber so dünnflüssig wie möglich ist. Es gibt auch sehr seltene Situationen, in denen ein Ballonkatheter nach der Deflation im Gefäß des Patienten festhängt. In so einem Fall kann sich der Arzt dazu entscheiden, um den Ballonkatheter entfernen zu können, den Ballonkatheter vorsätzlich im Gefäß zum Platzen zu bringen, um ihn dann entfernen zu können. In solch einem Fall darf einerseits kein Gas im Ballonkatheter vorhanden sein, da sich dieses in solch einem Fall spontan explosionsartig ausdehnen würde und das Gefäß zum Platzen bringen könnte. Zudem wäre in diesem Fall ein hochdosiertes Kontrastmittel der weiteren Gesundheit des Patienten abträglich.

### Zusammenfassung der Offenbarung

Es ist demnach die Aufgabe der vorliegenden Offenbarung, die Nachteile aus dem Stand der Technik zu vermeiden und eine Inflationsvorrichtung zur Inflation eines Ballonkatheters zur Angioplastie bereitzustellen, mit der ein für die Inflation erforderlicher Druck aufgebaut werden kann und bei der die Gefahr einer Fehlanwendung ausgeschlossen oder zumindest verringert werden kann.

Die Aufgabe der vorliegenden Offenbarung wird durch eine Inflationsvorrichtung mit den Merkmalen des Patentanspruchs 1 gelöst. Vorteilhafte Weiterbildungen sind Gegenstand der Unteransprüche.

Demnach wird die Aufgabe der vorliegenden Offenbarung durch eine Inflationsvorrichtung zur Inflation eines Ballonkatheters zur Angioplastie, mit einer Spritze, gelöst. Die Spritze weist einen hohl ausgebildeten Spritzenkörper (Spritzenzylinder), eine an den Spritzenkörper angeschlossene, mit dem Ballonkatheter (d.h. einem Ballonkatheterlumen) verbindbare Spritzenöffnung und einen in dem Spritzenkörper angeordneten Spritzenkolben auf.

Der Spritzenkolben begrenzt zusammen mit einer Spritzenkörperwandung einen mit der Spritzenöffnung verbundenen Druckraum der Spritze. Das heißt, dass ein innerhalb der Spritzenkörperwandung ausgebildetes Spritzenvolumen durch den Spritzenkolben in den Druckraum und einen Restraum/Leerraum (ein Restvolumen/Leervolumen) unterteilt wird. Zur Veränderung eines Volumens des Druckraums ist der Spritzenkolben axial in dem Spritzenkörper verlagerbar. Das heißt, dass der Druckraum je nach axialer Position des Spritzenkolbens unterschiedlich groß ist. Somit kann durch Verkleinerung des Druckraums ein Überdruck erzeugt werden, d.h. insbesondere in dem Druckraum enthaltene Flüssigkeit oder Luft komprimiert bzw. über die Spritzenöffnung aus der Spritze herausgefördert werden. Durch Vergrößerung des Druckraum kann ein Unterdruck erzeugt werden, d.h. insbesondere in dem Druckraum enthaltene Flüssigkeit oder Luft entspannt bzw. über die Spritzenöffnung in die Spritze eingesaugt werden.

Gemäß der Offenbarung ist die Spritze teilweise mit einem Kontrastmittelgemisch befüllt. Das heißt, dass eine (bestimmte) Menge des Kontrastmittelgemischs, d.h. eines in einem (bestimmten) Mischungsverhältnis verdünnten Kontrastmittels, in dem Druckraum enthalten ist und die Spritze nicht vollständig befüllt ist, so dass ein bestimmtes Leervolumen vorliegt. Der Spritzenkolben befindet sich demnach in einer mittleren Axialposition (keiner Endstellung), so dass er aus dieser Axialposition in beide Axialrichtung verlagerbar ist und dadurch sowohl das Kontrastmittelgemisch verdichtet und/oder aus dem Druckraum herausgefördert als auch insbesondere Luft in den Druckraum eingesaugt werden kann. Dabei ist die Spritze (bereits) vor der Inflation des Ballonkatheters und/oder in einem versiegelten Zustand der Spritze teilweise, d.h. nicht vollständig, befüllt/gefüllt. Ferner weist die Spritze gemäß der Offenbarung eine Kennzeichnung auf, welche Daten über die in dem Druckraum enthaltene Menge (/Füllmenge) des Kontrastmittelgemischs enthält. Zudem kann die Kennzeichnung vorzugsweise Daten über das Mischungsverhältnis des Kontrastmittelgemischs enthalten. Dabei enthält die Kennzeichnung Daten einer Füllmenge der vor der Inflation des Ballonkatheters und/oder in dem versiegelten Zustand teilweise gefüllten Spritze. Das heißt, dass die Kennzeichnung insbesondere angibt, zu welchem Teil die Spritze initial (teil-)gefüllt ist. Die Kennzeichnung kann vorzugsweise in Form eines maschinell lesbaren Codes, beispielsweise eines Barcodes, QR-Codes oder RFID-Transponders, ausgebildet sein.

Der Kern der Offenbarung liegt demzufolge darin, dass die Spritze bereits teilbefüllt, d.h. mit einer zum Druckaufbau im Ballonkatheter ausreichend großen Menge an Kontrastmittelgemisch und einem ausreichend großen Restziehvolumen, und gekennzeichnet für den Anwender zur Verfügung gestellt wird, so dass eine Fehlverwendung durch den Anwender beim Befüllen mit einer zu großen oder zu kleinen Menge (und vorzugsweise zusätzlich mit einem ungeeigneten Mischungsverhältnis) bei der Auswahl der teilbefüllten Spritze ausgeschlossen werden kann. Dem Anwender wird demnach nicht nur der Befüllungsschritt der Spritze, bei dem das unverdünnte Kontrastmittel vorzugsweise auf das richtige Mischungsverhältnis verdünnt und die richtige Menge mit der Spritze aufgezogen werden muss, sondern gleichzeitig die Identifizierung ebenjener Daten abgenommen, so dass die Benutzung möglichst unanfällig für Fehler ist. Durch die Verwendung eines maschinell lesbaren Codes bietet sich zusätzlich die Möglichkeit, die Auswahl zu überprüfen oder automatisiert zu unterstützen.

Im Gegensatz zu anderen Spritzenanwendungen, wie beispielsweise einer Medikamentenzuführung, ist es bei der Inflation von Ballonkathetern nicht vorwiegend entscheidend, dass die Flüssigkeitszuführung über die Spritze genau dosiert erfolgen und zu jedem Zeitpunkt gewährleistet sein muss, um eine Fehldosierung oder ein schlagartiges Beenden der Flüssigkeitszuführung zur Patientensicherheit zu vermeiden. Dagegen ist es bei der Inflation von Ballonkathetern zwingend erforderlich, dass die enthaltene Menge an Kontrastmittelgemisch ausreichend groß, aber auch ausreichend klein ist, um einerseits das erforderliche Restziehvolumen zur Entlüftung bereitstellen und andererseits eine schnelle Entleerung des zuvor dem Ballonkatheter zugeführten Kontrastmittelgemischs durchführen zu können. Somit ist die Anwendung zur Inflation von Ballonkathetern nicht mit anderen Spritzenanwendungen vergleichbar, bei denen das Restziehvolumen keine Bedeutung hat und daher die vorbefüllten Spritzen aus Kostengründen üblicherweise vollständig befüllt sind.

Als Kontrastmittel kann beispielsweise lopromid, lodixanol, loxaglate, lohexol, lopamidol, lomeprol, lomeron, Gadodiamide oder Gadolinium verwendet werden. Zur Verdünnung wird das Kontrastmittel vorzugsweise mit einer Kochsalzlösung, z.B. NaCl 0,9%, verdünnt. Dabei kann Gadodiamide oder Gadolinium auch unverdünnt verwendet werden. Als Mischungsverhältnis ist ein Verhältnis von Kontrastmittel zu Verdünnungsmittel von 1:1 bis 1:3, vorzugsweise von etwa 1:2, geeignet.

Gemäß einer bevorzugten Ausführungsform kann die Spritzenöffnung einen versiegelten Verschluss aufweisen. Somit kann dem Fall vorgebeugt werden, dass die Daten der Kennzeichnung zu dem Zeitpunkt, an dem der Verschluss (noch) versiegelt ist, nicht mit der tatsächlichen Füllmenge in der Spritze übereinstimmen. Dies hat den Vorteil, dass es nach dem Befüllen der Spritze mit dem Kontrastmittelgemisch und dem Versiegeln nicht möglich ist, die Befüllung zu verändern (insbesondere einen Teil des Kontrastmittelgemischs zu entnehmen), ohne dass dies ersichtlich ist. Folglich kann eine versehentliche Falschverwendung einer beispielsweise bereits (über die Anfangsfüllmenge hinaus) teilentleerten Spritze ausgeschlossen werden. Zudem kann eine Verunreinigung der Spritze oder der Befüllung verhindert werden.

Gemäß einer bevorzugten Ausführungsform kann die Spritze als ein Einwegprodukt (/Einmalprodukt/Wegwerfprodukt) ausgebildet sein. Das heißt, dass die Spritze als solche, u.a. aus Gründen der Sterilität und Patientensicherheit, nicht für die Wiederaufbereitung und zur Mehrfachverwendung (für verschiedene Patienten) geeignet ist. Die Spritze kann jedoch für die Inflation mehrerer Ballonkatheter hintereinander, beispielsweise für nacheinander eingesetzte, unterschiedlich große Ballonkatheter, eingesetzt werden.

Gemäß einer bevorzugten Ausführungsform kann die Spritze, vor der Inflation des Ballonkatheters und/oder in dem versiegelten Zustand der Spritze, zu 10 bis 80 %, vorzugsweise zu 15 bis 70 %, weiter bevorzugt zu 20 bis 60 %, besonders bevorzugt zu 25 bis 50 %, eines Gesamtvolumens des Spritzenkörpers mit dem Kontrastmittelgemisch befüllt sein. Die Spritze kann einen Spritzendurchmesser von 5 bis 50 Millimeter, vorzugsweise von 15 bis 30 Millimeter, haben. Ferner kann die Spritze ein Gesamtfüllvolumen, d.h. eine maximale Füllmenge (bei vollständig ausgezogenem/ausgefahrenem Spritzenkolben), von 10 bis 250 Milliliter, vorzugsweise von 20 bis 100 Milliliter, haben. Zudem kann die Menge des Kontrastmittelgemischs beispielsweise 5 bis 50 Milliliter, vorzugsweise 10 bis 25 Milliliter, betragen. Diese Größen und Füllmengen haben sich bei der Inflation von Ballonkathetern zur Angioplastie als besonders geeignet erwiesen.

Gemäß einer bevorzugten Ausführungsform kann die Kennzeichnung Daten eines Spritzentyps der Spritze enthalten. Beispielsweise können die Spritzentypdaten produktspezifische Daten sein, die für die Verwendung der Spritze relevant sind, wie ein (Gesamt-)Füllvolumen, eine Chargennummer, eine Artikelnummer und/oder ein maximal zulässiger Druck. Somit kann die korrekte Verwendung der Spritze vereinfacht werden, die Gefahr für Fehlverwendungen verringert werden und die Benutzung der Inflationsvorrichtung teilautomatisiert oder automatisiert werden. Die Spritze kann eine einzige Kennzeichnung (/Identifikationscodes), in der die Daten über den Spritzentyp und das Kontrastmittelgemisch gemeinsam enthalten sind, oder mehrere (z.B. zwei) einzelne Kennzeichnungen (/Identifikationscodes), der denen die Daten über den Spritzentyp und das Kontrastmittelgemisch separat voneinander enthalten sind, aufweisen. Beispielsweise können die separaten Kennzeichnungen auch auf unterschiedliche Weise lesbar sein.

Gemäß einer bevorzugten Ausführungsform kann die Inflationsvorrichtung einen, insbesondere in dem Spritzenkolben angeordneten, Drucksensor (/Druckstempel) zur Erfassung des Drucks in dem Druckraum aufweisen. Gemäß einer alternativen bevorzugten Ausführungsform kann der Druckraum mit einem Drucksensor (/einem separaten Messinstrument) zur Erfassung des Drucks in dem Druckraum, insbesondere über einen mit der Spritzenöffnung verbundenen Anschluss, vorzugsweise in Form eines 3-Wege-Hahns, verbindbar sein. Das heißt, dass der Druck in dem Druckraum nicht nur über ein manuelles Manometer angezeigt und direkt abgelesen, sondern gemessen und vorzugsweise automatisch weiterverarbeitet werden kann. Dadurch lassen sich Ablesefehler durch einen Benutzer vermeiden. Zudem kann insbesondere zwischen dem (externen) Drucksensor und dem Druckraum ein Sterilfilter angeordnet sein, um eine Verunreinigung des Kontrastmittelgemischs zu vermeiden.

Gemäß der bevorzugten Ausführungsform kann die Inflationsvorrichtung eine Steuervorrichtung aufweisen, mit welcher der Drucksensor zur Übertragung des erfassten Drucks kabellos, beispielsweise per Funk, oder kabelgebunden verbindbar ist. Somit kann der Druck ausgelesen werden und vorzugsweise für die automatische Steuerung weiterverarbeitet werden.

Gemäß der bevorzugten Ausführungsform kann die Inflationsvorrichtung eine Druckbegrenzungsvorrichtung zur Begrenzung eines maximalen Drucks in dem Druckraum aufweisen. Vorzugsweise kann die Druckbegrenzungsvorrichtung derart ausgebildet sein, dass sie ein Verkleinern des Druckraums verhindert, sobald der Druck in dem Druckraum den maximal zulässigen Druck der Spritze erreicht. Beispielsweise kann die Druckbegrenzungsvorrichtung durch Hemmung einen Überdruck verhindern, insbesondere wenn der am Drucksensor gemessenen Druck den maximal zulässigen Druck, der aus der Kennzeichnung auslesbar ist, erreicht. So kann vorzugsweise automatisch/automatisiert eine Fehlverwendung verhindert werden.

Gemäß einer bevorzugten Ausführungsform kann die Inflationsvorrichtung einen Positionssensor zur Erfassung einer Position des Spritzenkolbens aufweisen. Dies hat den Vorteil, dass der Füllstand/Füllgrad der Spritze (und damit die aus dem Druckraum herausgeförderte Menge an Kontrastmittelgemisch) zusätzlich überwacht werden kann. Ferner kann dadurch ein Verlagern des Spritzenkolbens in seine Endlagen vermieden werden.

Gemäß einer bevorzugten Ausführungsform kann die Inflationsvorrichtung eine Antriebseinheit aufweisen, die mechanisch mit dem Spritzenkolben gekoppelt oder koppelbar ist, um den Spritzenkolben zur Verkleinerung des Druckraums und zur Vergrößerung des Druckraums axial zu verlagern. Die Antriebseinheit kann derart ausgebildet und mit dem Spritzenkolben gekoppelt sein, dass sie eine Kraftverstellung und eine Schnellverstellung realisiert. Bei der Kraftverstellung können Drücke von 6 bis 30 bar auf den Spritzenkolben aufgebracht werden. Beispielsweise kann die Kraftverstellung durch Drehung des Spritzenkolbens, welche etwa mittels eines Gewindes in eine Axialverschiebung gewandelt wird, erfolgen. Eine Kraftverstellung ist insbesondere zur Inflation selbst, d.h. zur Zuführung des Kontrastmittelgemischs in den Ballonkatheter unter hohem Druck, erforderlich. Das heißt, dass insbesondere ein Vorfahren des Spritzenkolbens/Verkleinern des Druckraums mit hoher Kraft /(deutlich) gegenüber der Schnellverstellung erhöhter Kraft erfolgen kann. Bei der Schnellverstellung können Geschwindigkeiten von mindestens einer Spritzenverstelllänge/Sekunde erreicht werden. Beispielsweise kann die Schnellverstellung durch Lösen eines Gewindeeingriffs erfolgen. Eine Schnellverstellung ist insbesondere zur Entleerung des Ballonkatheters und zur Entlüftung des Ballonkatheters erforderlich. Das heißt, dass insbesondere ein Zurückfahren des Spritzenkolbens/Vergrößern des Druckraums mit hoher Geschwindigkeit/(deutlich) gegenüber der Kraftverstellung erhöhten Geschwindigkeit erfolgen kann. Mit anderen Worten ist die Antriebseinheit vorzugsweise so ausgebildet, dass sie eine kraftaufbringende Komponente und eine Komponente zum schnellen Verlagern aufweist.

Gemäß einer bevorzugten Ausführungsform kann die Inflationsvorrichtung mit einem Entlüftungsventil zur Entlüftung des Druckraums, insbesondere über einen oder den mit der Spritzenöffnung verbundenen Anschluss, vorzugsweise in Form eines oder des 3-Wege-Hahns, verbindbar sein. Somit kann die Entlüftung des Ballonkatheters besonders einfach dadurch erfolgen, dass zunächst eine Verbindung zwischen Spritzenöffnung und Ballonkatheter hergestellt wird, insbesondere durch Öffnung eines mit dem Ballonkatheter verbundenen Anschlusses des 3-Wege-Hahns, und der Spritzenkolben zurückgezogen wird, wodurch in dem Ballonkatheter enthaltene Luft aus dem Ballonkatheter heraus in den Druckraum gesaugt wird, und dann die Verbindung zwischen Spritzenöffnung und Ballonkatheter unterbrochen wird, insbesondere durch Sperrung des mit dem Ballonkatheter verbundenen Anschlusses des 3-Wege-Hahns, eine Verbindung zwischen Spritzenöffnung und Entlüftungsventil hergestellt wird, insbesondere durch Öffnung des mit dem Entlüftungsventil verbundenen Anschlusses des 3-Wege-Hahns, und der Spritzenkolben vorgeschoben wird, wodurch in dem Druckraum enthaltene Luft aus dem Druckraum heraus über das Entlüftungsventil zur Umgebung hin abgegeben wird. Dieser Entlüftungsvorgang kann auch mehrfach bis zur vollständigen Entlüftung des Ballonkatheters durchgeführt werden. Anschließend kann die Inflation durch Zuführung des Kontrastmittelgemischs in den Ballonkatheter erfolgen.

Alternativ kann die Entlüftung des Ballonkatheters dadurch erfolgen, dass eine Verbindung zwischen Spritzenöffnung und Ballonkatheter hergestellt wird und der Spritzenkolben zurückgezogen wird, wodurch in dem Ballonkatheter enthaltene Luft aus dem Ballonkatheter heraus in den Druckraum gesaugt wird, wobei die Luft bei der anschließenden Inflation des Ballonkatheters durch Zuführung des Kontrastmittelgemischs in dem Druckraum enthalten bleibt. Dabei wird das erneute Zuführen der in dem Druckraum enthaltenen Luft durch eine Lage der Spritze verhindert, indem die Spritze bei der Inflation mit der Spritzenöffnung nach unten gehalten wird, so dass sich die Luft nur in einem oberen Teil des Druckraums befindet und nur das in einem unteren Teil des Druckraums befindliche Kontrastmittelgemisch dem Ballonkatheter zugeführt wird.

Weiter alternativ kann die Entlüftung des Ballonkatheters dadurch erfolgen, dass eine Verbindung zwischen Spritzenöffnung und Ballonkatheter hergestellt wird und der Spritzenkolben zurückgezogen wird, wodurch in dem Ballonkatheter enthaltene Luft aus dem Ballonkatheter heraus in den Druckraum gesaugt wird, wobei die aus dem Ballonkatheter heraus in den Druckraum eingesaugte Luft über eine semipermeable Membran aus dem Druckraum abgeführt wird.

Gemäß einem weiteren Aspekt der Offenbarung, der vorzugsweise in Kombination, aber auch unabhängig von der teilbefüllten und gekennzeichneten Spritze vorliegen kann, kann die Inflationsvorrichtung einen mit der Spritzenöffnung verbundenen oder verbindbaren Ballonkatheter aufweisen. Der Ballonkatheter kann eine Kennzeichnung aufweisen, welche Daten eines Ballonkathetertyps des Ballonkatheters enthält. Die Kennzeichnung kann vorzugsweise in Form eines maschinell lesbaren Codes, beispielsweise eines Barcodes, QR-Codes oder RFID-Transponders, ausgebildet sein. Beispielsweise können die Ballonkathetertyp produktspezifische Daten sein, die für die Verwendung des Ballonkatheters relevant sind, wie ein Entlüftungsvolumen, ein Maximalinflationsvolumen, eine Druck-Inflationsdurchmesser-Relation, eine vorgesehene Anwendung oder ein für die vorgesehene Anwendung bestimmtes Mischungsverhältnis an Kontrastmittelgemisch, eine Chargennummer und/oder eine Artikelnummer. Somit kann die korrekte Verwendung des Ballonkatheters vereinfacht werden, die Gefahr für Fehlverwendungen verringert werden und die Benutzung der Inflationsvorrichtung teilautomatisiert oder automatisiert werden.

Gemäß einer bevorzugten Ausführungsform kann die Inflationsvorrichtung ausgebildet sein, um in Abhängigkeit des Ballonkathetertyps und der enthaltenen Menge des Kontrastmittelgemischs oder der Spritzenkolbenposition zu erkennen, ob die Spritze zur vollständigen Inflation des Ballonkatheters und/oder zur Entlüftung des Ballonkatheters geeignet ist. Zur Inflation des Ballonkatheters ist je nach Ballonkathetertyp eine bestimmte Mindestmenge des Kontrastmittelgemischs erforderlich, um den Ballonkatheter bis auf den gewünschten/vorgegebenen Ziel-Durchmesser aufdehen zu können. Zudem ist zur Entlüftung des Ballonkatheters je nach Ballonkathetertyp ein bestimmtes Mindestleervolumen der Spritze erforderlich, um überhaupt erst durch Zurückziehen des Spritzenkolbens die Luft aus dem Ballonkatheter heraussaugen zu können und insbesondere bei fehlender Möglichkeit der Druckraumentlüftung den Ballonkatheter vollständig entlüften zu können. Somit kann einfach erkannt werden, ob die gewählte, bereits teilbefüllte Spritze für den ausgewählten Ballonkatheter geeignet ist, und dadurch die Verwendung vereinfacht oder teilautomatisiert/automatisiert werden.

Gemäß einer bevorzugten Ausführungsform kann die Inflationsvorrichtung ausgebildet sein, um in Abhängigkeit des Ballonkathetertyps und des Mischungsverhältnisses des Kontrastmittelgemischs zu erkennen, ob die Spritze zur vorgesehenen Anwendung des Ballonkatheters geeignet ist. Zur Gewährleistung geeigneter röntgendichter Eigenschaften ist je nach Ballonkathetertyp ein bestimmtes Mischungsverhältnis des Kontrastmittelgemischs erforderlich. Somit kann einfach erkannt werden, ob die Röntgeneigenschaften der gewählten, bereits teilbefüllten Spritze für den ausgewählten Ballonkatheter geeignet sind, und dadurch die Verwendung vereinfacht oder teilautomatisiert/automatisiert werden.

Mit anderen Worten betrifft die Offenbarung eine Inflationsvorrichtung, die aus zwei Komponenten, nämlich einer elektromechanischen Grundeinheit und einem Einwegprodukt in Form einer Spritze mit Druckkörper/Kolben, aufgebaut ist. Die Grundeinheit weist einen mechanischen Antrieb auf, durch den der Kolben innerhalb eines (Spritzen-)Zylinders/Körpers verschoben werden kann. Durch die aufgebrachte Kraft kann der erzeugte Druck ermittelt werden. Der Druck kann über die Kraft am Kolben gesteuert werden. Zusätzlich kann eine Lagemessung vorhanden sein, um die Position des Kolbens innerhalb des Zylinders/Körpers zu erfassen. Dadurch kann ein Füllgrad der Spritze überwacht und ein Verschieben des Kolbens in seine Endlagen vermieden werden. Zudem kann ein, etwa über einen integrierten 3-Wege-Hahn, mit der Spritze verbindbarer oder verbundener Drucksensor vorhanden sein, mit dem der Druck in der Spritze gemessen und/oder der gemessene/ermittelte Druck korrigiert werden kann. Die Spritze kann einen produktspezifischen QR-Code oder Barcode aufweisen, der an der Grundeinheit eingelesen werden kann, so dass ein entsprechendes Produkt ausgewählt wird. Zudem oder alternativ kann das entsprechende Produkt über Bedienelemente der Grundeinheit ausgewählt werden. Ferner kann ein, etwa über den integrierten 3-Wege-Hahn, mit der Spritze verbindbares oder verbundenes Ventil vorhanden sein, mit dem die Leitungen entlüftet bzw. im Notfall/Fehlerfall, z.B. bei Stromausfall, geöffnet werden können. Der 3-Wege-Hahn kann sich an der Spitze/Öffnung der Einwegeinheit/Spritze befinden, um den Anschluss des Katheters und der Druckmesseinheit und des Ventils in die Grundeinheit zu ermöglichen.

Vorzugsweise kann die Einwegeinheit/Spritze mit Kontrastmittel befüllt geliefert werden, um gleiche Eigenschaften bezüglich Füllmenge und Röntgensichtbarkeit zu gewährleisten, wobei ein Befüllen und Entleeren des Katheters dann nach konstanten rheologischen Eigenschaften erfolgt. Der Kolben kann mit einem Durchmesser von 5 bis 50 mm, vorzugsweise von 15 bis 30 mm, ausgeführt sein. Ein maximaler Hub setzt sich aus einer Füllmenge von 10 bis 250 ml, vorzugsweise von 20 bis 100 ml, zusammen. Eine Füllung des Einwegprodukts kann zwischen 5 und 50 ml, vorzugsweise 10 bis 25 ml, mit Kontrastmittel betragen.

Zum Entlüften der Einwegeinheit/Spritze wird der 3-Wege-Hahn so gedreht, dass das Spritzenvolumen mit dem Katheterlumen verbunden ist und der Kolben (maximal) herausgezogen. Dann wird der Kolben schnell entlastet, d.h. in weniger als 5 Sekunden, vorzugsweise in weniger als 2 Sekunden, so dass das Kontrastmittel in das Katheterlumen geleitet wird. Nach der Entlüftung müssen hohe Drücke aufgebracht werden, für die eine adäquate Auslegung der Mechanik notwendig ist. Zudem ist eine schnelle Verschiebung erforderlich, was üblicherweise hohen Kräften entgegensteht.

Somit sind die Grundfunktionen (und Komponenten) der Inflationsvorrichtung das Erzeugen eines Unterdrucks zum Entlüften des Kathetersystems, das Erreichen und Halten eines (produktspezifischen) Überdrucks, das Einstellen eines passenden Drucks, das Ermöglichen einer Not- bzw. Schnellentleerung des Katheters (Not-Aus), das Erreichen eines Mindestdrucks von 6 atm, das Erreichen eines Höchstdrucks von 30 atm, das Bereitstellen einer vorgefüllten Spritze mit 10 bis 30 ml Kontrastmittel.

Alternativ können/kann der Drucksensor und/oder das Ventil in der Grundeinheit entfallen und/oder der 3-Wege-Hahn in der Einwegeinheit entfallen. Die Einwegeinheit bzw. die Grundeinheit kann schwenkbar ausgeführt sein und manuell oder automatisch gedreht werden, um eine Wiederverwendung der Einwegeinheit durch Entlüftung des Spritzenkolbens zu ermöglichen. Ein Katheter darf hierbei nicht angeschlossen sein.

### Kurzbeschreibung der Figuren

Fig. 1 ist eine schematische Darstellung eines Grundprinzips einer Inflationsvorrichtung gemäß der vorliegenden Offenbarung;
Fig. 2 ist eine schematische Darstellung eines Querschnitts der Inflationsvorrichtung aus Fig. 1;
Fig. 3 ist eine schematische Darstellung einer bevorzugten Ausführungsform der Inflationsvorrichtung gemäß der vorliegenden Offenbarung; und
Fig. 4 ist eine schematische Darstellung eines Querschnitts der Inflationsvorrichtung aus Fig. 3.

Nachstehend werden Ausführungsformen der vorliegenden Offenbarung auf der Basis der zugehörigen Figuren beschrieben.

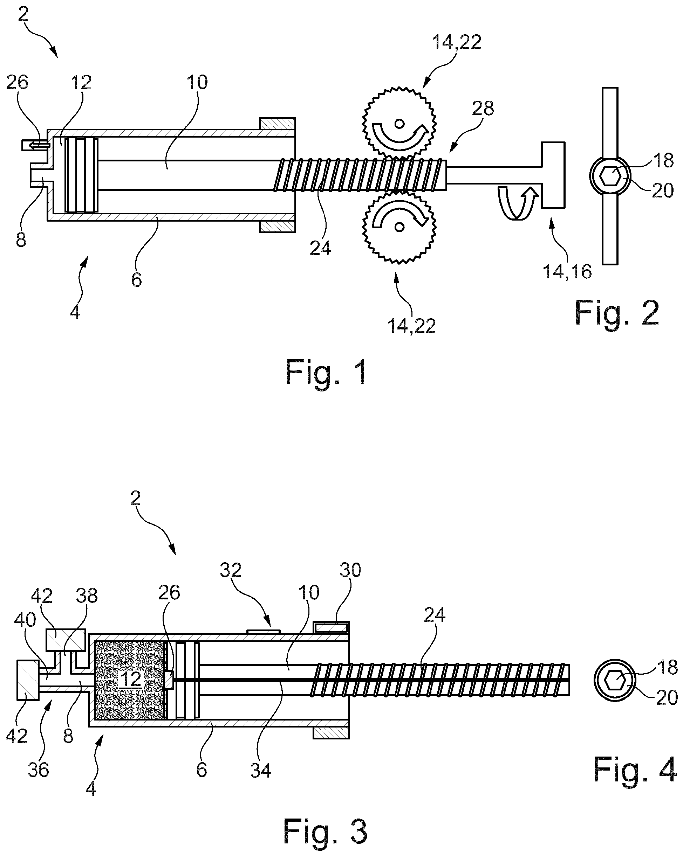

Fig. 1 zeigt ein Grundprinzip einer Inflationsvorrichtung 2 gemäß der vorliegenden Offenbarung. Die Inflationsvorrichtung 2 dient zur Inflation eines (nicht dargestellten) Ballonkatheters zur Angioplastie.

Die Inflationsvorrichtung 2 weist eine Spritze 4 auf. Die Spritze 4 weist einen hohl ausgebildeten Spritzenkörper/Spritzenzylinder 6 auf. Zudem weist die Spritze 4 eine an den Spritzenkörper 6 angeschlossene, mit dem Ballonkatheter verbindbare Spritzenöffnung 8 auf. Ferner weist die Spritze 4 einen in dem Spritzenkörper 6 angeordneten Spritzenkolben 10 auf. Der Spritzenkolben 10 begrenzt zusammen mit einer Spritzenkörperwandung einen mit der Spritzenöffnung 8 verbundenen Druckraum 12. Der Druckraum 12 ist vorzugsweise über eine Dichtung, hier in Form von zwei Dichtungsringen, zwischen der Spritzenkörperwandung und dem Spritzenkolben 10 abgedichtet. Der Spritzenkolben 10 ist zur Veränderung eines Volumens des Druckraums 12 (Spritzenvolumens) axial in dem Spritzenkörper 6 verlagerbar. Vorzugsweise kann die Spritze 4 als ein Einwegprodukt ausgebildet sein.

Die Inflationsvorrichtung 2 weist eine Antriebseinheit 14 auf. Die Antriebseinheit 14 ist mechanisch mit dem Spritzenkolben 10 gekoppelt, um den Spritzenkolben 10 axial zu verlagern. Die Antriebseinheit 14 weist eine drehbare/drehantreibbare Antriebswelle 16 auf, deren Rotation mit einer Rotation des Spritzenkolbens 10 (um seine Längsachse) gekoppelt ist. In der dargestellten Ausführungsform weist die Antriebswelle 16 dazu eine Formschlussgeometrie 18, hier in Form eines Außensechskants, auf, der in eine entsprechende, am Spritzenkolben 10 ausgebildete Gegenformschlussgeometrie 20, hier in Form eines Innensechskants, eingreift (vgl. Fig. 2), so dass ein Drehmoment von der Antriebswelle 16 formschlüssig auf den Spritzenkolben 10 übertragen werden kann.

Zudem weist die Antriebseinheit 14 zwei bezüglich des Spritzenkolbens 10 gegenüberliegend angeordnete Zahnräder 22 auf. Die Zahnräder 22 stehen im Verzahnungseingriff mit einem an dem Spritzenkolben 10 ausgebildeten (Außen-) Gewinde 24 (bzw. sind in Verzahnungseingriff bringbar). Die Zahnräder 22 sind bezüglich der Axialrichtung des Spritzenkolbens 10 fest angebracht, so dass der Spritzenkolben 10, etwa nach Art eines Schneckengetriebes, bei Drehung um seine Längsachse (durch die Antriebswelle 16) und/oder bei Drehung der Zahnräder 22 um ihre Längsachse aufgrund des Verzahnungseingriffs mit den Zahnrädern 22 axial verlagert wird.

Somit kann der Spritzenkolben 10 durch Drehung der Antriebswelle 16 in die eine Drehrichtung ausgefahren (und damit der Druckraum 12 vergrößert) werden und durch Drehung der Antriebswelle 16 in die andere Drehrichtung eingefahren (und damit der Druckraum 12 verkleinert) werden. Bei gleichzeitiger Drehung eines oder beider Zahnräder 22 entgegen der durch die Antriebswelle 16 aufgebrachten Kraft entsprechenden Richtung, d.h. bei aktivem Antrieb zumindest eines Zahnrads 22, kann die Axialbewegung des Spritzenkolbens 10 beschleunigt werden. Bei gleichzeitigem Stillstand eines oder beider Zahnräder 22, d.h. beim Bremsen zumindest eines Zahnrads 22, kann der Spritzenkolben 10 mit hoher Kraft bewegt werden. Zudem kann der Spritzenkolben 10 durch Drehung der Zahnräder 22 in die eine Drehrichtung ausgefahren (und damit der Druckraum 12 vergrößert) werden und durch Drehung der Zahnräder 22 in die andere Drehrichtung eingefahren (und damit der Druckraum 12 verkleinert) werden. Bei gleichzeitiger Drehung entgegen der durch die Zahnräder 22 aufgebrachten Kraft entsprechenden Richtung oder beim Stillstand der Antriebswelle 16 kann die Axialbewegung des Spritzenkolbens 10 beschleunigt werden.

Zudem kann die Inflationsvorrichtung 2 einen an den Druckraum 12 angeschlossenen Sensor 26 zur Messung einer Reaktionskraft aufweisen, durch den ein Druck in dem Druckraum 12 messbar bzw. ermittelbar ist.

Ferner kann die Inflationsvorrichtung 2 einen in Fig. 1 lediglich angedeuteten Positionssensor 28 zum Erfassen einer Position des Spritzenkolbens 10 aufweisen.

Fig. 3 zeigt eine bevorzugte Ausführungsform der Inflationsvorrichtung 2 gemäß der vorliegenden Offenbarung, deren Aufbau dem in Bezug auf Fig. 1 beschriebenem Grundprinzip entspricht. Eine Darstellung der Antriebsvorrichtung 14 ist in der schematischen Darstellung der Fig. 3 weggelassen.

Die Spritze 4 ist teilweise mit einem Kontrastmittelgemisch befüllt. Das Kontrastmittelgemisch ist in dem Druckraum 12 enthalten. Vorzugsweise kann die Spritze zu 10 bis 80 %, vorzugsweise zu 15 bis 70 %, weiter bevorzugt zu 20 bis 60 %, besonders bevorzugt zu 25 bis 50 %, eines Gesamtvolumens des Spritzenkörpers 6 mit dem Kontrastmittelgemisch befüllt sein. Das heißt, dass ein Restvolumen/Restziehvolumen, in dem kein Kontrastmittelgemisch enthalten ist, vorhanden ist und die Spritze im Bereich des Druckraums 12 gefüllt und im Bereich des Restvolumens leer ist. Der Spritzenkolben 10 befindet sich demnach in einer Position zwischen seinen Endlagen.

Die Spritze 4 weist eine Kennzeichnung 30 auf, welche Daten über eine in dem Druckraum 12 enthaltene Menge des Kontrastmittelgemischs und/oder ein Mischungsverhältnis des Kontrastmittelgemischs enthält. Die Kennzeichnung 30 ist in der dargestellten Ausführungsform auf einer Außenseite des Spritzenkörpers 6 aufgebracht. Vorzugsweise ist die Kennzeichnung 30 in Form eines maschinell lesbaren Codes, beispielsweise eines Barcodes, QR-Codes oder RFID-Transponders, ausgebildet. In der dargestellten Ausführungsform ist die Kennzeichnung 30 in Form des RFID-Transponders ausgebildet, über den die Menge des Kontrastmittelgemischs, d.h. der Füllmenge der vor der Inflation des Ballonkatheters und/oder in dem versiegelten Zustand teilweise gefüllten Spritze 4, sowie vorzugsweise ein Mischungsverhältnis des Kontrastmittelgemischs eindeutig identifizierbar sind.

Die Spritze 4 weist eine weitere Kennzeichnung 32 auf, welche Daten eines Spritzentyps der Spritze enthält. Die Kennzeichnung 32 ist in der dargestellten Ausführungsform auf einer Außenseite des Spritzenkörpers 6 aufgebracht. Vorzugsweise ist die Kennzeichnung 32 in Form eines maschinell lesbaren Codes, beispielsweise eines Barcodes, QR-Codes oder RFID-Transponders, ausgebildet. In der dargestellten Ausführungsform ist die Kennzeichnung 32 in Form des CR-Codes ausgebildet. Daten des Spritzentyps sind beispielsweise ein (Gesamt-)Füllvolumen der Spritze 2, eine Chargennummer, eine Artikelnummer und/oder ein maximal zulässiger Druck.

Zudem kann die Spritzenöffnung einen (nicht explizit dargestellten) versiegelten Verschluss aufweisen.

In der in Fig. 3 dargestellten Ausführungsform ist der Drucksensor 26 als ein Druckstempel ausgebildet, der an einer dem Druckraum zugewandten Seite des Spritzenkolbens 10 angeordnet ist. Der Drucksensor 26 ist über eine Datenleitung 34, etwa von einer (nicht dargestellten) Steuereinheit auslesbar. Alternativ kann der Drucksensor 26 kabellos, etwa über Funk, auslesbar sein.

Die Spritze 4 weist einen 3-Wege-Hahn 26 auf. Ein erster Anschluss des 3-Wege-Hahns 26 ist mit der Spritzenöffnung 8 verbunden.

Ein zweiter Anschluss 38 des 3-Wege-Hahns 26 ist mit einem (externen) Drucksensor und/oder einem Entlüftungsventil verbindbar oder verbunden. Ein dritter Anschluss 40 des 3-Wege-Hahns 26 ist mit dem Ballonkatheter verbindbar oder verbunden. Der zweite Anschluss 38 und der dritte Anschluss 40 können beispielsweise in Form eines Luer-Adapters/Luer-Verschlusses 42 ausgebildet sein.

Insbesondere erfolgt eine Entlüftung des Ballonkatheters dadurch, dass zunächst eine Verbindung zwischen Spritzenöffnung 8 und Ballonkatheter hergestellt wird, insbesondere durch Öffnung des dritten Anschlusses 40, und der Spritzenkolben 10, vorzugsweise schnell, zurückgezogen wird. Dadurch wird in dem Ballonkatheter enthaltene Luft aus dem Ballonkatheter heraus in den Druckraum 12 gesaugt. Dann wird die Verbindung zwischen Spritzenöffnung 8 und Ballonkatheter unterbrochen, insbesondere durch Sperrung des dritten Anschlusses 40, eine Verbindung zwischen Spritzenöffnung 8 und Entlüftungsventil wird hergestellt, insbesondere durch Öffnung des zweiten Anschlusses 38, und der Spritzenkolben 10 wird vorgeschoben. Dadurch wird in dem Druckraum enthaltene Luft aus dem Druckraum 12 heraus über das Entlüftungsventil zur Umgebung hin abgegeben. Dieser Entlüftungsvorgang kann auch mehrfach bis zur vollständigen Entlüftung des Ballonkatheters durchgeführt werden.

Insbesondere erfolgt eine Inflation des Ballonkatheters dadurch, dass (nach dem Entlüften des Ballonkatheters) eine Verbindung zwischen Spritzenöffnung 8 und Ballonkatheter hergestellt wird, insbesondere durch Öffnung des dritten Anschlusses 40, und der Spritzenkolben 10, vorzugsweise mit hoher Kraft, vorgeschoben wird. Dabei können Drücke von 6 bis 30 bar aufgebaut werden und der Ballonkatheter entsprechend einer ballonkatheterspezifischen Druck-Inflationsdurchmesser-Relation auf einen gewünschten Ziel-Durchmesser aufgedehnt werden.

Insbesondere erfolgt eine Deflation des Ballonkatheters dadurch, dass (nach der Inflation des Ballonkatheters und dadurch erfolgten Aufweitung eines Blutgefäßes) der Spritzenkolben 10, vorzugsweise schnell, zurückgezogen wird. Dadurch wird das zuvor dem Ballonkatheter zugeführte Kontrastmittelgemisch aus dem Ballonkatheter heraus in den Druckraum 12 gesaugt und der Durchmesser des Ballonkatheters verkleinert, so dass er aus dem Blutgefäß herausgezogen werden kann.

Dabei ist es möglich, dass der Spritzenkolben 10 zunächst schnell zurückgezogen wird, um den Patienten schnell von dem Druck zu entlasten. Nach der schnellen Entlastung kann sich noch ein Restvolumen in dem Ballonkatheter befinden. Anschließend kann der Spritzenkolben 10 weiter, beispielsweise langsam, zurückgezogen werden, um das Restvolumen/den Restdruck in dem Ballonkatheter aktiv leerzuziehen und den Durchmesser des deflatierten Ballonkatheters so klein wie möglich zu machen, so dass er möglichst einfach aus dem Patienten entfernt/herzausgezogen werden kann.

## Patentansprüche

1. Inflationsvorrichtung (2) zur Inflation eines Ballonkatheters zur Angioplastie, mit
einer Spritze (4), die einen hohl ausgebildeten Spritzenkörper (6), eine an den Spritzenkörper (6) angeschlossene, mit dem Ballonkatheter verbindbare Spritzenöffnung (8) und einen in dem Spritzenkörper (6) angeordneten Spritzenkolben (10) aufweist, der zusammen mit einer Spritzenkörperwandung einen mit der Spritzenöffnung (8) verbundenen Druckraum (12) begrenzt und zur Veränderung eines Volumens des Druckraums (12) axial in dem Spritzenkörper (6) verlagerbar ist,
**dadurch gekennzeichnet, dass**
die Spritze (4) vor der Inflation des Ballonkatheters und/oder in einem versiegelten Zustand teilweise mit einem Kontrastmittelgemisch befüllt ist und eine Kennzeichnung (30, 32), vorzugsweise in Form eines maschinell lesbaren Codes aufweist, welche Daten über eine in dem Druckraum (12) enthaltene Füllmenge der vor der Inflation des Ballonkatheters und/oder in dem versiegelten Zustand teilweise gefüllten Spritze sowie vorzugsweise ein Mischungsverhältnis des Kontrastmittelgemischs enthält.

2. Inflationsvorrichtung (2) nach Anspruch 1, **dadurch gekennzeichnet, dass** die Spritzenöffnung (8) einen versiegelten Verschluss aufweist.

3. Inflationsvorrichtung (2) nach Anspruch 1 oder 2, **dadurch gekennzeichnet, dass** die Spritze (4), insbesondere vor der Inflation des Ballonkatheters und/oder in dem versiegelten Zustand, zu 10 bis 80 %, vorzugsweise zu 15 bis 70 %, weiter bevorzugt zu 20 bis 60 %, besonders bevorzugt zu 25 bis 50 %, eines Gesamtvolumens des Spritzenkörpers (6) mit dem Kontrastmittelgemisch befüllt ist.

4. Inflationsvorrichtung (2) nach einem der Ansprüche 1 bis 3, **dadurch gekennzeichnet, dass** die Kennzeichnung (30, 32) Daten eines Spritzentyps der Spritze enthält, vorzugsweise über ein Füllvolumen, eine Chargennummer, eine Artikelnummer und/oder einen maximal zulässigen Druck.

5. Inflationsvorrichtung (2) nach einem der Ansprüche 1 bis 4, **dadurch gekennzeichnet, dass** die Inflationsvorrichtung (2) einen, insbesondere in dem Spritzenkolben (10) angeordneten, Drucksensor (26) zur Erfassung des Drucks in dem Druckraum (12) aufweist oder der Druckraum (12) mit einem Drucksensor zur Erfassung des Drucks in dem Druckraum (12), insbesondere über einen mit der Spritzenöffnung (8) verbundenen Anschluss (38), vorzugsweise in Form eines 3-Wege-Hahns (36), verbindbar ist.

6. Inflationsvorrichtung (2) nach Anspruch 5, **dadurch gekennzeichnet, dass** die Inflationsvorrichtung (2) eine Steuervorrichtung aufweist, mit welcher der Drucksensor (26) zur Übertragung des erfassten Drucks kabellos, beispielsweise per Funk, oder kabelgebunden verbindbar ist, und/oder dass die Inflationsvorrichtung (2) eine Druckbegrenzungsvorrichtung zur Begrenzung eines maximalen Drucks in dem Druckraum (12) aufweist.

7. Inflationsvorrichtung (2) nach einem der Ansprüche 1 bis 6, **dadurch gekennzeichnet, dass** die Inflationsvorrichtung (2) einen Positionssensor (28) zur Erfassung einer Position des Spritzenkolbens (10) aufweist.

8. Inflationsvorrichtung (2) nach einem der Ansprüche 1 bis 7, **dadurch gekennzeichnet, dass** die Inflationsvorrichtung (2) eine Antriebseinheit (14) aufweist, die mechanisch mit dem Spritzenkolben (10) gekoppelt oder koppelbar ist, um den Spritzenkolben (10) zur Verkleinerung des Druckraums (12) und zur Vergrößerung des Druckraums (12) axial zu verlagern, wobei die Antriebseinheit (14) derart ausgebildet und mit dem Spritzenkolben (10) gekoppelt ist, dass sie eine Kraftverstellung zur Aufbringung von Drücken mit 6 bis 30 bar und eine Schnellverstellung realisiert.

9. Inflationsvorrichtung (2) nach einem der Ansprüche 1 bis 8, **dadurch gekennzeichnet, dass** die Inflationsvorrichtung (2) mit einem Entlüftungsventil zur Entlüftung des Druckraums (12), insbesondere über einen oder den mit der Spritzenöffnung (8) verbundenen Anschluss (38), vorzugsweise in Form eines oder des 3-Wege-Hahns (36), verbindbar ist.

10. Inflationsvorrichtung (2) nach einem der Ansprüche 1 bis 9, **dadurch gekennzeichnet, dass** die Inflationsvorrichtung (2) einen mit der Spritzenöffnung (8) verbundenen oder verbindbaren Ballonkatheter aufweist, der eine Kennzeichnung, vorzugsweise in Form eines maschinell lesbaren Codes, beispielsweise eines Barcodes, QR-Codes oder RFID-Transponders, aufweist, welche Daten eines Ballonkathetertyps des Ballonkatheters enthält, vorzugsweise über ein Entlüftungsvolumen, ein Maximalinflationsvolumen, eine Druck-Inflationsdurchmesser-Relation, eine vorgesehene Anwendung oder ein für die vorgesehene Anwendung bestimmtes Mischungsverhältnis an Kontrastmittelgemisch, eine Chargennummer und/oder eine Artikelnummer,
wobei die Inflationsvorrichtung (2) vorzugsweise ausgebildet ist, um in Abhängigkeit des Ballonkathetertyps und der enthaltenen Menge des Kontrastmittelgemischs oder der Spritzenkolbenposition zu erkennen, ob die Spritze (4) zur vollständigen Inflation des Ballonkatheters und/oder zur Entlüftung des Ballonkatheters geeignet ist, und/oder um in Abhängigkeit des Ballonkathetertyps und des Mischungsverhältnisses des Kontrastmittelgemischs zu erkennen, ob die Spritze (4) zur vorgesehenen Anwendung des Ballonkatheters geeignet ist.

## Claims

1. An inflation device (2) for inflating a balloon catheter for angioplasty, comprising
a syringe (4), which has a hollow syringe body (6), a syringe opening (8) connected to the syringe body (6) and connectable to the balloon catheter and a syringe plunger (10) arranged in the syringe body (6), which together with a syringe-body wall delimits a pressurized room (12) connected to the syringe opening (8) and is axially displaceable in the syringe body (6) in order to change a volume of the pressurized room (12),
**characterized in that**
the syringe (4), before inflation of the balloon catheter and/or in a sealed state, is partially filled with a contrast agent mixture and has a labeling (30, 32), preferably in the form of a machine-readable code, which contains data about a filling quantity contained in the pressurized room (12) of the syringe partially filled before inflation of the balloon catheter and/or in the sealed state, and/or preferably a mixing ratio of the contrast agent mixture.

2. The inflation device (2) according to claim 1, **characterized in that** the syringe opening (8) has a sealed closure.

3. The inflation device (2) according to claim 1 or 2, **characterized in that** the syringe (4), in particular before inflation of the balloon catheter and/or in the sealed state, is filled with the contrast agent mixture to 10 to 80 %, preferably to 15 to 70 %, more preferably to 20 to 60 %, particularly preferably to 25 to 50 %, of a total volume of the syringe body (6).

4. The inflation device (2) according to any of claims 1 to 3, **characterized in that** the labeling (30, 32) contains data of a syringe type of the syringe, preferably about a filling volume, a batch number, an article number and/or a maximum permissible pressure.

5. The inflation device (2) according to any of claims 1 to 4, **characterized in that** the inflation device (2) comprises a pressure sensor (26) for detecting the pressure in the pressurized room (12), the pressure sensor (26) being arranged in particular in the syringe plunger (10), or that the pressurized room (12) is connectable with a pressure sensor for detecting the pressure in the pressurized room (12), in particular via a terminal (38) connected to the syringe opening (8), preferably in the form of a three-way stopcock (36).

6. The inflation device (2) according to claim 5, **characterized in that** the inflation device (2) has a control device with which the pressure sensor (26) is connectable wirelessly, for example by radio, or is connectable by cable for transmitting the detected pressure, and/or **in that** the inflation device (2) has a pressure limitation device for limiting a maximum pressure in the pressurized room (12).

7. The inflation device (2) according to any of claims 1 to 6, **characterized in that** the inflation device (2) comprises a position sensor (28) for detecting a position of the syringe plunger (10).

8. The inflation device (2) according to any of claims 1 to 7, **characterized in that** the inflation device (2) has a drive unit (14) that is or can be coupled mechanically to the syringe plunger (10) in order to axially displace the syringe plunger (10) to reduce the pressurized room (12) and to enlarge the pressurized room (12), the drive unit (14) being configured and coupled to the syringe plunger (10) such that it realizes a force adjustment for applying pressures of 6 to 30 bar and a quick adjustment.

9. The inflation device (2) according to any of claims 1 to 8, **characterized in that** the inflation device (2) is connectable to a vent valve for venting the pressurized room (12), in particular via a terminal or the terminal (38) connected to the syringe opening (8), preferably in the form of a three-way stopcock or the three-way stopcock (36).

10. The inflation device (2) according to any of claims 1 to 9, **characterized in that** the inflation device (2) has a balloon catheter connected or connectable to the syringe opening (8), which has a labeling, preferably in the form of a machine-readable code, for example, a barcode, QR code or RFID transponder, that contains data of a balloon catheter type of the balloon catheter, preferably about a venting volume, a maximum inflation volume, a pressure-inflation diameter relation, an intended application or a mixing ratio of a contrast agent mixture intended for the intended application, a batch number and/or an article number,
wherein the inflation device (2) preferably is configured to recognize, depending on the type of balloon catheter and the quantity of the contrast agent mixture contained, or the syringe plunger position, whether the syringe (4) is suitable for complete inflation of the balloon catheter and/or for venting of the balloon catheter, and/or to recognize, depending on the type of balloon catheter and the mixing ratio of the contrast agent mixture, whether the syringe (4) is suitable for the intended application of the balloon catheter.

## Revendications

1. Dispositif de gonflage (2) pour gonfler un cathéter à ballonnet destiné à l'angioplastie, avec
une seringue (4) qui présente un corps de seringue (6) conçu de manière creuse, une ouverture de seringue (8) raccordée au corps de seringue (6) pouvant être reliée au cathéter à ballonnet, et un piston de seringue (10) disposé dans le corps de seringue (6) qui délimite, conjointement avec une paroi de corps de seringue, une chambre de pression (12) reliée à l'ouverture de seringue (8) et qui peut être déplacée axialement dans le corps de seringue (6) pour modifier un volume de la chambre de pression (12),
**caractérisé en ce que**
la seringue (4) est partiellement remplie d'un mélange de produit de contraste avant le gonflage du cathéter à ballonnet et/ou dans un état scellé et présente un marquage (30, 32), de préférence sous forme d'un code lisible par machine qui contient des données relatives à une quantité de remplissage contenue dans la chambre de pression (12) de la seringue partiellement remplie avant le gonflage du cathéter à ballonnet et/ou dans l'état scellé, ainsi que de préférence un rapport de mélange du mélange de produit de contraste.

2. Dispositif de gonflage (2) selon la revendication 1, **caractérisé en ce que** l'ouverture de la seringue (8) présente une fermeture scellée.

3. Dispositif de gonflage (2) selon la revendication 1 ou 2, **caractérisé en ce que** la seringue (4), en particulier avant le gonflage du cathéter à ballonnet et/ou dans l'état scellé, est remplie de 10 à 80 %, de préférence de 15 à 70 %, plus préférentiellement de 20 à 60 %, le plus préférentiellement de 25 à 50 %, d'un volume total du corps de seringue (6) avec le mélange de produit de contraste.

4. Dispositif de gonflage (2) selon l'une quelconque des revendications 1 à 3, **caractérisé en ce que** le marquage (30, 32) contient des données d'un type de seringue de la seringue, de préférence relatives à un volume de remplissage, un numéro de lot, un numéro d'article et/ou une pression admissible maximale.

5. Dispositif de gonflage (2) selon l'une quelconque des revendications 1 à 4, **caractérisé en ce que** le dispositif de gonflage (2) présente un capteur de pression (26) disposé en particulier dans le piston de seringue (10) pour détecter la pression dans la chambre de pression (12) ou **en ce que** la chambre de pression (12) peut être reliée à un capteur de pression pour détecter la pression dans la chambre de pression (12), en particulier par l'intermédiaire d'un raccord (38) relié à l'ouverture de seringue (8), de préférence sous forme d'un robinet à 3 voies (36).

6. Dispositif de gonflage (2) selon la revendication 5, **caractérisé en ce que** le dispositif de gonflage (2) présente un dispositif de commande auquel le capteur de pression (26) peut être relié sans fil, par exemple par radio, ou par câble, pour transmettre la pression détectée, et/ou **en ce que** le dispositif de gonflage (2) présente un dispositif de limitation de pression pour limiter une pression maximale dans la chambre de pression (12).

7. Dispositif de gonflage (2) selon l'une quelconque des revendications 1 à 6, **caractérisé en ce que** le dispositif de gonflage (2) présente un capteur de position (28) pour détecter une position du piston de seringue (10).

8. Dispositif de gonflage (2) selon l'une quelconque des revendications 1 à 7, **caractérisé en ce que** le dispositif de gonflage (2) présente une unité d'entraînement (14) qui est couplée ou peut être couplée mécaniquement au piston de seringue (10) pour déplacer axialement le piston de seringue (10) afin de réduire la chambre de pression (12) et d'agrandir la chambre de pression (12), dans lequel l'unité d'entraînement (14) est conçue et couplée au piston de seringue (10) de sorte qu'elle réalise un réglage de force pour appliquer des pressions de 6 à 30 bars et un réglage rapide.

9. Dispositif de gonflage (2) selon l'une quelconque des revendications 1 à 8, **caractérisé en ce que** le dispositif de gonflage (2) peut être relié à une soupape de purge pour purger la chambre de pression (12), en particulier par l'intermédiaire d'un raccord (38) relié à l'ouverture de seringue (8), de préférence sous forme d'un robinet à 3 voies (36).

10. Dispositif de gonflage (2) selon l'une quelconque des revendications 1 à 9, **caractérisé en ce que** le dispositif de gonflage (2) présente un cathéter à ballonnet relié ou pouvant être relié à l'ouverture de seringue (8) qui présente un marquage, de préférence sous forme d'un code lisible par machine, par exemple un code-barres, un code QR ou un transpondeur RFID, marquage qui contient des données d'un type de cathéter à ballonnet du cathéter à ballonnet, de préférence relatives à un volume de purge, un volume de gonflage maximal, un rapport pression-diamètre de gonflage, une application prévue ou un rapport de mélange de produit de contraste déterminé pour l'application prévue, un numéro de lot et/ou un numéro d'article,
dans lequel le dispositif de gonflage (2) est de préférence conçu pour reconnaître, en fonction du type de cathéter à ballonnet et de la quantité de produit de contraste contenue ou de la position de piston de seringue, si la seringue (4) est adaptée au gonflage complet du cathéter à ballonnet et/ou à la purge du cathéter à ballonnet, et/ou pour reconnaître, en fonction du type de cathéter à ballonnet et du rapport de mélange du mélange de produit de contraste, si la seringue (4) est adaptée à l'utilisation prévue du cathéter à ballonnet.
